# EUROPEAN PATENT SPECIFICATION

(11) **EP 4 464 235 B1**
(45) Date of publication and mention of the grant of the patent: **26.11.2025**
(21) Application number: 23305800.7
(22) Date of filing: 17.05.2023
(51) Int. Cl.: A61B 3/028, G02C 7/02, A61B 3/18

(54) **DEVICE AND METHOD FOR DETERMINING A FINAL VALUE OF A VISION CORRECTION POWER OF A CORRECTIVE LENS**
VORRICHTUNG UND VERFAHREN ZUR BESTIMMUNG EINES ENDGÜLTIGEN WERTS EINER SEHKORREKTURLEISTUNG EINER KORREKTURLINSE
DISPOSITIF ET PROCÉDÉ DE DÉTERMINATION D'UNE VALEUR FINALE D'UNE PUISSANCE DE CORRECTION DE VISION D'UNE LENTILLE CORRECTRICE

(43) Date of publication of application: 20.11.2024
(73) Proprietor: Essilor International, 94220 Charenton-Le-Pont (FR)
(72) Inventor: JORET, Paul, 94220 Charenton-le-Pont (FR); MARIN, Gildas, 75012 Paris (FR); LONGO, Adèle, 75020 Paris (FR); HERNANDEZ-CASTANEDA, Martha, 75012 Paris (FR); GRAND-CLEMENT, Didier, 93100 Montreuil (FR)
(74) Representative: Jacobacci Coralis Harle

(56) References cited:
- EP-A1- 4 129 155
- WO-A1-2022/013231
- US-A1- 2022 095 910
- US-A1- 2023 139 007
- US-B2- 11 224 338

## Description

### TECHNICAL FIELD OF THE INVENTION

The invention relates to a device for determining a final value of a vision correction power of a corrective lens to be worn by an individual.

### BACKGROUND INFORMATION AND PRIOR ART

In order to provide a visual equipment adapted to the visual defect of an individual, it is necessary to assess this visual defect and determine the features of an equipment adapted to correct it.

In practice, an individual's vision may be tested during a subjective test protocol, using a phoropter allowing placing successively in front of his eye lenses to provide different test values of said vision correction power to each of the eyes of the individual.

This test protocol may be achieved using a classical phoropter using distinct successive lenses with different values of power or a phoropter having one or more lenses of continuously variable power.

At each trial of the subjective test, corresponding to a current lens placed in front of his eye, the individual is asked to express a visual assessment that corresponds to an indication of a preferred visual state among two visual states presented to him or if he cannot decide between the two. The two visual states may correspond to his vision of two different images through the current lens or may correspond to his vision through the previous lens and his vision through the current lens, for example.

Many protocols for achieving such a subjective test are known. A standard device for determining a final value of a vision correction power of a corrective lens to be placed in front of an eye of an individual is described in the documents US 2023/139007 A1 and US 2022/095910 A1

At the end of such a subjective refraction determination process, a preliminary value of a visual correction power for a potential corrective lens for improving the visual performance of the individual is determined.

However, this preliminary value is usually modified by the eye care professional, based on his empiric experience, to determine a final value of the visual correction power that is adapted to the individual, in particular ensuring that the individual accepts the vision correction lens and adapts easily to it.

The final value therefore depends on the eye-care professional involved. It is not determined in an accurate and reproducible way.

### SUMMARY OF THE INVENTION

One object of the invention is to provide a new device adapted to provide a final value of the vision correction power in an accurate and reproducible way, to be used directly, without further modification, for manufacturing a vision correction lens of a vision correction equipment. This will ensure a quick adaptation of the individual to the vision correction lens as well as an efficient improvement of the visual performance of the individual.

The above objects are achieved according to the invention by a device for determining a final value of a vision correction power of a final corrective lens to be placed in front of an eye of an individual, said device comprising a computer with one or more memories and one or more processors, wherein:
- said one or more memories have in memory:
   - an initial measured value of a magnitude representative of a refractive feature of an eye of the individual, measured using a first optometry instrument,
   - an estimated value of said magnitude, calculated using a predetermined model of a second optometry instrument,
   - a current value of said magnitude determined based on a current corrective lens previously worn by the individual,
- said one or more processors are programmed to achieve the following steps:
   a) comparing a difference between the measured value and the current value of said magnitude to a first threshold value, and
   b) when the difference between the measured value and the current value of said magnitude is greater than or equal to said first threshold value, comparing the difference between said measured value and said estimated value of said magnitude to a second threshold value,
   c) determining said final value of the vision correction power based on said comparisons.

Thanks to the device according to the invention, the final value determined may take into account the measured value of the magnitude, refractive feature of the eye of the individual, as it is usually the case for determining a vision correction power, but also the current value of the vision correction power and an estimated value corresponding to a determination with a different optometry instrument, in order to determine a final value of the correction power.

The final value is reproducibly and efficiently produced, without relying on the knowledge and skills of the operator to adapt the measured value to the individual. The final value is determined to be prescribed directly without further empirical modification.

Other advantageous non limiting features of the system according to the invention are listed hereafter:
- said one or more memories have in memory data relative to at least one relevant personal parameter relative to said individual and said first and second threshold value are determined taking into account said data;
- said relevant personal parameter comprises at least one of age, gender, sensitivity to a variation of said vision correction power, kind of ametropia, features of a previous vision correction equipment used, one or more values of a refractive feature of the eye determined through an objective method, type of complaints of said individual relative to his previous vision correction equipment, design of the current/final vision correction lens;
- said relevant personal parameters are determined statistically on the basis of a database comprising data relative to a set of personal parameters and corresponding one or more measured values of a vision correction power of a lens and/or one or more values of a refractive feature of an eye determined with first and second optometry instruments, a current value of vision correction power provided by a current corrective lens worn by the individual and/or a current value of said refractive feature, for a group of test individuals, and preferred value of the vision correction power chosen by each test individual, by determining correlations between personal parameters of said set and the preferred value of vision correction power;
- said magnitude representative of a refractive feature of an eye of the individual is determined taking into account said data relative to at least one relevant personal parameter relative to said individual;
- said first and second thresholds are determined statistically based on said database, by determining correlations between the values of said relevant personal parameters and the preferred value of vision correction power;
- in step c), when the difference between the measured value and the current value of said magnitude is less than said first threshold value, the one or more processors are programmed to determine the final value of vision correction power as equal to a measured vision correction power corresponding to the measured value of said magnitude;
- in step c), when the difference between said measured value and said estimated value of said magnitude is greater than or equal to the second threshold value, the one or more processors are programmed to determine the final value of vision correction power as equal to an estimated vision correction power corresponding to the estimated value of said magnitude or a modified value determined to be between a measured vision correction power corresponding to the measured value of said magnitude and said estimated vision correction power,
- said one or more memories having in memory:
- a measured value of a magnitude representative of another refractive feature of the eye of the individual, measured using said first optometry instrument,
- an estimated value of said magnitude representative of another refraction feature of said eye of the individual, calculated using said model of the second optometry instrument,

in step c), when the difference between said measured value and said estimated value of said magnitude representative of another refractive feature is less than the second threshold value, the one or more processors are programmed to compare the difference between said measured value and said estimated value of said magnitude representative of said other refraction feature to a third threshold value and
determine said final value of the vision correction power taking into account this comparison.
   - when the difference between said measured value and said estimated value of said magnitude representative of said other refraction feature is less than said third threshold value, the final value of the vision correction power is determined to be equal to a vision correction power corresponding to said estimated value of said magnitude representative of the other refractive feature or a modified value determined to be between a vision correction power corresponding to said measured value of said magnitude representative of the other refractive feature and said estimated value of this magnitude;
   - when the difference between said measured value and said estimated value of said magnitude representative of said other refractive feature is greater than or equal to said third threshold value, the final value of the vision correction power is determined to be equal to a vision correction power corresponding to said measured value of this magnitude;
   - a sensitivity of the individual to a variation of said vision correction power refractive feature is recorded in said one or more memories and said one or more processors are programmed to take into account said sensitivity when determining the final value;
   - said model of said second optometry instrument is determined using a regression algorithm or a learning algorithm trained on another database comprising the following data for a reference group of individual : values of said magnitude determined for each test subject of a group of test subjects by said second optometry instrument, associated with the values of : the test lenses of each trials of the subjective test and the corresponding answers of the test subject;
   - said one or more memories have in memory measured values of a plurality of refraction features of an eye of the individual and/or corresponding vision correction powers, measured using a first optometry instrument ; estimated values of said plurality of refraction features of the eye of the individual and/or of the corresponding vision correction powers, calculated using a model of a second optometry instrument, and current values of said refractive features of the eye and/or of said vision correction powers of a current corrective lens previously worn by the individual;
   - each refractive feature and/or corresponding vision correction power of said plurality of refractive features and vision correction powers being associated with specific first, second and third threshold values and said one or more processors being programmed to achieve steps a) to c) for each refractive feature of said plurality of refractive features, taking into account the associated threshold values;
   - said refractive feature comprises at least one of the following: sphere, cylinder, spherical equivalent power, addition, J0, J45.

The invention also relates to a method for determining a final value of a vision correction power of a final corrective lens to be place in front of en eye of an individual, comprising the following steps:
- recording in one or more memories of a computer:
   - an initial measured value of a magnitude representative of a refractive feature of the eye of the individual, measured using a first optometry instrument, said refractive feature corresponding to said vision correction power,
   - an estimated value of said magnitude, calculated using a predetermined model of a second optometry instrument,
   - a current value of said magnitude determined based on a current corrective lens previously worn by the individual,
- thanks to one or more processors of said computer:
   a) comparing a difference between the measured value and the current value of said magnitude to a first threshold value, and
   b) when the difference between the measured value and the current value of said magnitude is greater than or equal to said first threshold value, comparing the difference between said measured value and said estimated value of said magnitude to a second threshold value,
   c) determining said final value of the vision correction power based on said comparisons.

### DETAILED DESCRIPTION OF EXAMPLE(S)

The following description with reference to the accompanying drawings will make it clear what the invention consists of and how it can be achieved. The invention is not limited to the embodiment/s illustrated in the drawings. Accordingly, it should be understood that where features mentioned in the claims are followed by reference signs, such signs are included solely for the purpose of enhancing the intelligibility of the claims and are in no way limiting on the scope of the claims.

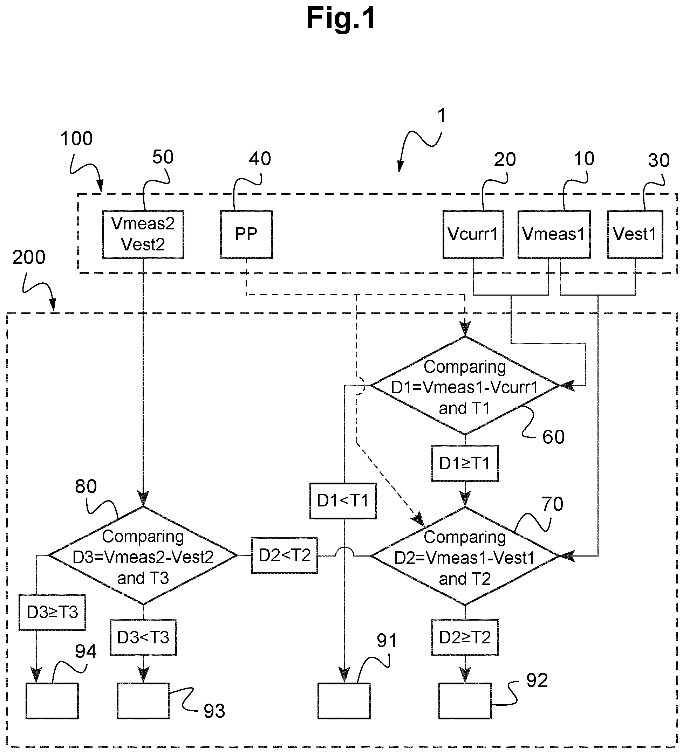

In the accompanying drawings, the single Figure 1 is a schematic view of an optometry system according to the invention, comprising one or more memories and one or more processors, overlaid with a block diagram of steps of the method according to the invention.

The invention relates to a device 1 for determining a final value of a vision correction power of a final corrective lens to be worn by an individual. It is adapted to perform a method according to the invention for determining a final value of a vision correction power of a final corrective lens to be worn by an individual.

Such a device 1 is schematically represented on the single appended figure 1. Said device 1 comprising a computer with one or more memories 100 and one or more processors 200.

The appended single figure 1 shows the steps of an embodiment of the method of the invention overlaid with the part of the device used to implement them.

Said one or more memories 100 of the device 1 have in memory:
- an initial measured value of a magnitude representative of a refractive feature of an eye of the individual, measured using a first optometry instrument,
- an estimated value of said magnitude, calculated using a predetermined model of a second optometry instrument,
- a current value of said magnitude determined based on a current corrective lens previously worn by the individual,

These values are inputted in said one or more memories 100. The method of the invention correspondingly comprises the following steps:
- recording (block 10 of the single figure) in said one or more memories said measured value Vmeas1 of said magnitude,
- recording (block 30 of the single figure) in said one or more memories said estimated value Vest1 of said magnitude,
- recording (block 20 of the single figure) in said one or more memories said current value Vcurr1 of said magnitude.

The magnitude representative of the refractive feature of the eye of the subject may be for example the refractive feature itself or a vision correction power for a vision correction lens determined to improve the visual performance of the individual by correcting a visual defect linked to this refractive feature.

In the first case, the initial measured value of said magnitude is then equal to an initial measured refractive feature of an eye of the individual, measured using said first optometry instrument. The estimated value of said magnitude is equal to an estimated value of said refractive feature of the eye calculated using a predetermined model of a second optometry instrument, and the current value of said magnitude is a current value of said refractive feature, determined based on the current corrective lens worn by the individual.

In the second case, the initial measured value of said magnitude is then equal to an initial measured vision correction power, measured using said first optometry instrument. The estimated value of said magnitude is equal to an estimated value of said vision correction power calculated using a predetermined model of a second optometry instrument, and the current value of said magnitude is a current value of said vision correction power of the current corrective lens worn by the individual.

The vision correction power of the vision correction lens adapted to correct a given refractive feature of an eye of the individual is determined based on the refractive feature and takes into account the position of the vision correction lens in front of the eye. In particular, it takes into account the distance between said vision correction lens and the eye. It may also take into account pantoscopic tilt and wrap angles of the lens in front of the eye. Said first and second optometry instrument may be programmed to provide either a measured value of the refractive feature or a measured value of the vision correction power when used.

The model of said second optometry instrument may therefore consequently be built to provide one of these measured values or the other.

The vision correction power of the corrective lens to be worn by the individual in front of one his eyes comprises one or more of the following: a spherical power also called sphere, a cylindrical power, also called cylinder and/or a cylindrical axis, a spherical equivalent power with J0 and J45, a prism power and/or a prism axis, difference in cylinder, J0 or J45 between the right and left lenses, said vision correction power corresponding to zones of near or far vision of the corrective lens. It may also comprise an addition, a rotation of the cylinder axis between previously worn equipment and the one under consideration.

The spherical equivalent power is calculated by adding the sum of the spherical power with half of the cylindrical power.

The corresponding refractive features of the eye of the individual to be corrected may comprise a dioptric value relative to a spherical refraction value, a cylindrical refraction value and /or axis; a prism refraction value, a difference between the spherical refraction of the eyes, said refractive feature being measured in far and/or near vision conditions.

In practice, said magnitude may comprise any of the above listed refractive feature of the eye of the individual and corresponding vision correction power of the vision correction lens to be placed in front of this eye.

The first optometry instrument comprises a phoropter allowing placing successively in front of his eye lenses to provide different test values of said vision correction power to each of the eyes of the individual. The first optometry instrument is used to perform a subjective test protocol.

This first optometry instrument comprises for example a classical phoropter, for example with stack of trial lenses of predefined fixed power by step of 0.25D, or a phoropter having lenses of continuously variable power. It is preferably a phoropter with lenses of continuously variable power or mirror or LFD (Light Field Display) or moving distance such as Badal or handheld system, able to provide successive test values of the vision correction power.

At each trial of the subjective test, corresponding to a current lens placed in front of his eye, the individual is asked to express a visual assessment that corresponds to an indication of a preferred visual state among two visual states presented to him or if he cannot decide between the two. The two visual states may correspond to his vision of two different images through the current lens, for example during a duochrome test, or may correspond to his vision through the previous lens and his vision through the current lens, for example.

Specific types of visual targets are used for each subjective test protocol.

Many protocols for achieving such a subjective test are known.

At the end of such a subjective refraction determination process, the initial value of said magnitude is determined, in particular an initial value of a visual correction power for the corrective lens for improving the visual performance of the individual or an initial value of the refractive feature of the eye of the individual is determined. The initial value of said magnitude takes into account the answers of the individual during the test. It may also take into account experimental parameters used during the test, such as the distance between the eye of the individual and each test lens, the pantoscopic tilt, the wrap angle, the object distance, luminance and contrast (to derive the pupil size for example).

The initial value of said magnitude is determined in order to obtain a maximum visual acuity after vision correction of the eye. No other parameter is taken into account. The initial value of the vision correction power usually aims at maximizing the visual acuity of the individual wearing an optical equipment with the corrective lens determined, without taking into account other parameters or information about the individual.

The first optometry instrument is preferably a high precision device allowing a determination of the initial value of the vision correction power using a vision power increment between two successive trials of the subjective test protocol strictly lower than 0.25 diopter, for example comprised between 0.05 and 0.2 diopter.

The first optometry instrument moreover preferably takes into account the sensitivity of the individual to a change in the vision correction power considered and the continuous variation of the power of each test lens allows adjustments adapted to the individual.

The first optometry instrument also comprises a display unit to display the visual targets used in the subjective test to the individual. The visual targets preferably comprise details, blurring and different depths. It comprises for example fine details, smoothed details, depth cues, texture and astigmatism sensitive features, that is to say, oriented features.

In an embodiment, the device 1 according to the invention may comprise said first optometry instrument. The subjective test is performed with the first optometry instrument and said measured value of said magnitude is determined by said first optometry instrument and recorded in said one or more memories. To this end the device according to the invention may comprise communication means adapted to receive data from the first optometry instrument.

Alternatively, the first optometry instrument may be distinct and optionally distant from the device of the invention. The device according to the invention may comprise communication means adapted to receive data from the first optometry instrument.

In any case, the device according to the invention may comprise an inputting device to input the measured value of said magnitude into said one or more memories.

The second optometry instrument comprises for example a phoropter using a vision power increment different from the vision power increment used by the first optometry instrument. The second optometry instrument uses for example the usual vision power increment of 0.25 diopter between two successive trials of the subjective test. It is then different from the first optometry instrument. The second optometry instrument may comprise a classical phoropter or a phoropter with continuously variable power. The second optometry instrument may be in practice the first optometry instrument used with different settings, for example a different vision power increment value.

The predetermined model of said second optometry instrument is built using a first database aggregating data from the achievement and results of subjective test performed with the second optometry instrument in a network of optometry clinics. It comprises values of said magnitude determined for each test subject of a group of test subjects by said second optometry instrument, associated with the values of: the test lenses of each trials of the subjective test and the corresponding answers of the test subject.

It may also comprise data such as: pupillary distance, lens eye distance, screen distance, visual acuity targeted, visual acuity measured, subjective preference for a given power, response time during the subjective refraction test, visual acuity measured or subjective feedback obtained from a patient such as preference for a given power on a given target, set-up parameters of the phoropter such as eye lens distance, pupillary distance, screen distance. Such data could be recorded by an automated phoropter.

Said first database may also comprise: age, gender, previous equipment optical formula, refractometer measurements, plaints, final refraction and final prescription, monocular accommodative facility, amplitude of accommodation, in facti measurements related to near vision comfort understanding and eyestrain.

These data could lead to further train the algorithm and could later be used as input to refine the model. Said predetermined model of said second optometry instrument is determined using a regression algorithm or a learning algorithm trained on said first database.

Said learning algorithm may comprise machine learning or deep learning algorithm. The predetermined model is for example calculated based on said first database, by a Convolutional Neural Network method (CNN) or by a Partial Least Square method. Any other method known of the man skilled in the art may also be applied.

Said predetermined model provides an estimated value of said magnitude determined by said second optometry instrument by inputting entry data comprising: any of the data listed in the first database. It may for example comprise age, optical features of the previously worn equipment, optical design characterized as soft progressive addition lens, hard progressive addition lens, commercial name of progressive addition lens, mid distance (i.e. an intermediary distance ranging from 50 cm to 1,5 m), commercial name of mid distance, automatic refractometer measurements including aberrometer or retinoscopy measurements.

When said magnitude is the vision correction power of the vision correction lens, said measured value and estimated value of said magnitude comprise at least one of the following, preferably all of the following, spherical power, cylindrical power and axis of the vision correction lens. It may also comprise addition, spherical equivalent power, J0, J45 at one or several points of the lens.

When said magnitude is the of the eye refractive feature, said measured value and estimated value of said magnitude comprise at least one of the following, preferably all of the following ; spherical refraction, cylindrical refraction and axis of the eye, difference in cylinder, J0 or J45 between the eyes, said refractive features being measured in near or far vision conditions. It may also comprise an addition, a level of anisometropia, a rotation of the cylinder axis between a previous and current measurement, accommodation amplitude, punctum proximum.

The estimated value may be calculated at any time and inputted into said device of the invention. It may also be retrieved from a database.

The current value of said magnitude is typically determined based on the current optical equipment of the individual. The current value of the vision correction power of a current corrective lens worn by the individual corresponds typically to the value of the vision correction power of the lens used by the individual at the time of performing the subjective test. A corresponding current value of the refractive feature of the eye may be deduced thereof. The individual removes his optical equipment for performing the subjective test. The current value of the vision correction power may be measured directly on this optical equipment and inputted in said device. Alternatively, the current value of the magnitude may be supplied by the individual or retrieved from a database or previous prescription for the individual or from an identification card of the current equipment of the individual.

Said one or more processors of said computer are programmed to achieve the following steps:
a) comparing (block 60 of the single figure) a difference D1 between the measured value Vmeas1 of the refractive feature and the current value Vcurr1 of said magnitude to a first threshold value T1, and
b) when the difference between the measured value and the current value of the magnitude is greater than or equal to said first threshold value, in other words when D1 = Vmeas1-Vcurr1 ≥ T1, comparing (block 70 of the single figure) the difference D2 between said measured value Vmeas1 and said estimated value Vest1 to a second threshold value T2,
c) determining said final value of the vision correction power based on said comparisons.

Preferably, said one or more memories have in memory data relative to at least one relevant personal parameter relative to said individual. Said first and second threshold value are determined taking into account said data.

Said magnitude representative of a refractive feature of an eye of the individual is also determined taking into account said data relative to at least one relevant personal parameter relative to said individual.

In other words, the type of magnitude, that is to say the type of refractive feature or vision correction power taken into account by said device and method according to the invention is statistically determined conjointly with the relevant personal parameters of the individual. The statistical analysis allows to identify the refractive feature and/or vision correction power, the relevant personal parameter and the corresponding thresholds that should be used together to efficiently determine the final vision correction power for said individual.

To this end, said method of the invention correspondingly comprises recording (block 40 of the single figure) in said one or more memories said data relative to at least one relevant personal parameter PP relative to said individual.

Said relevant personal parameter comprises at least one of : age, gender, sensitivity to a variation of said vision correction power, kind of ametropia, features of a previous vision correction equipment used, one or more values of a refractive feature of the eye of the individual determined through an objective method, type of complaints of said individual relative to his previous vision correction equipment, design of the current/final vision correction lens, the kind of activity practiced by the individual.

The age can be taken into account numerically or by using age categories such as kids, teens, adults, seniors. The age is for example taken into account as it implies different kind of designs for the vision correction lens.

The sensitivity to a variation of said vision correction power represents the ability of the eye of the individual to perceive a change in the vision correction power. It is for example quantified by the smallest variation of the vision correction power that the eye may distinguish. It is determined during the subjective refraction test performed with the first optometry instrument.

The kind of ametropia may comprise indications about the previous prescription of the individual, such as myopia, presbyopia, hyperopia, astigmatism, prismatic defects, phoria...

The features of a previous vision correction equipment used may for example comprise the value of the vison correction power of a lens used in a vision correction equipment previously used by the individual.

The one or more values of a refractive feature of an eye of the individual determined through an objective method comprise values of the refractive feature of the eye corresponding to the vision correction power of the lens. In other words this value quantifies the refractive feature of the eye which is corrected by said vision correction power. It is for example the value of the spherical refraction of the eye or the value of the cylindrical refraction or axis when the vision correction power is a sphere or a cylinder or axis. The objective method comprises a step of measuring this refractive feature of the eye with an objective optical instrument, such as an auto-refractometer.

The type of complaints may comprise side effects or appreciation of the efficiency/comfort of the previous equipment.

The design of the current and/or final vision correction lens may also be taken into account as the type of lens, favoring comfort or visual performance, may influence the adaptation of the individual to the current and/or final vision correction lens.

Said relevant personal parameters are determined statistically.

This is achieved on the basis of a second database comprising the following data, each determined for every member of a group of test individuals:
- data relative to a set of personal parameters,
- corresponding vision correction power values determined with first and second optometry instruments,
- current value of vision correction power provided by a current corrective lens worn by the individual, and
- preferred value of the vision correction power chosen by each test individual.

The second database may for example be obtained by asking said test individuals to wear a first optical equipment having vision correction lenses exhibiting the measured values of the vision correction powers during a first period of time, for example two weeks or a month, and a second optical equipment having vision correction lenses exhibiting the estimated values of the vision correction powers during a second period of time, preferably equal to the first period of time, then state the preferred optical equipment and the reasons why or rate each equipment or the difference between both globally or for particular criteria an situation from which is derived the preferred equipment. The second database may also comprise data obtained through immediate comparisons of the first and second optical equipment.

The preferred value is picked by the test individual among the vision correction power values determined with first and second optometry instruments and the current value of vision correction power provided by a current corrective lens worn by the individual.

To this end, correlations between personal parameters of said set and the preferred value of vision correction power are determined. This is achieved by a statistical treatment. Any appropriate statistical treatment may be considered. Linear discriminant analysis and C 4-5 are appropriate. C-PLS, PLS-DA, PLS-DA may also be used.

In particular, relevant magnitudes, defined as the one or more refractive features of the eye and/or the vision correction powers of the vision correction lens that are decisive in the acceptation or choice of the individual between a measured lens as a whole having all the measured values of the vision correction powers and an estimated lens as a whole having all the estimated values of the vision correction powers may be statistically determined. The set of parameters is preferentially kept as small as possible for robustness.

Said first and second thresholds are determined statistically based on said second database, by determining correlations between the values of said relevant personal parameters and the preferred value of vision correction power. This is represented schematically by hashed lines between block 40 and blocks 60 and 70 on the single figure.

Similarly, said relevant magnitudes are determined statistically based on said second database, by determining correlations between the values of said relevant personal parameters and the preferred value of vision correction power.

In an embodiment of the device according to the invention, said second database may be recorded in said one or more memories of said computer and said one or more processors may additionally be programmed to determine said first and second thresholds based on the statistical analysis of the second database described above. The method according to the invention may comprise the additional steps of determining said first and second thresholds by performing said statistical analysis of said second database.

The method according to the invention may also comprise the additional steps of determining said relevant magnitudes by performing said statistical analysis of said second database.

In another embodiment, said one or more memories may be provided with a relationship or a table linking values of the relevant personal parameters to the values of the first and second thresholds. the one or more processors may then be programmed to deduce from said relevant personal parameters the value of said first and second thresholds to achieve said comparisons steps 60, 70 (single figure) by using said relationship or said table.

Said one or more memories may be provided with a relationship or a table linking values of the relevant personal parameters to the relevant magnitudes.

In step c), when the difference D1 between the measured value Vmeas1 and the current value Vcurr1 of said magnitude is less than said first threshold value T1 during step a), in other words when D1= Vmeas1-Vcurr1 <T1, the one or more processors are programmed to determine the final value of vision correction power based on the measured value (block 91 of the single figure).

In practice, the one or more processors are programmed to determine the final value of vision correction power as equal to a measured vision correction power corresponding to the measured value of said magnitude.

In other words, when said magnitude is the vision correction power, the measured vision correction power is directly equal to the measured value of the magnitude.

When said magnitude is the refractive feature of the eye, the measured vision correction power is deduced from the measured value of the magnitude by determining the vision correction power of the vision correction lens adapted to improve the visual performance of the eye having the measured refractive feature. The distance between the vision correction lens and the eye is in particular taken into account.

In step c), when the difference D2 between said measured value Vmeas1 and said estimated value Vest1 of the magnitude is greater than or equal to the second threshold value T2, in other words when D2 = Vmeas1-Vest1 ≥ T2 during step b), the one or more processors are programmed to determine the final value of vision correction power based on the estimated value of said magnitude (block 92 of the single figure).

In practice, they are programmed to determine said final value as equal to an estimated vision correction power corresponding to the estimated value of said magnitude or to a modified value determined to be between a measured vision correction power corresponding to the measured value of said magnitude and said estimated vision correction power.

When said magnitude is the vision correction power, the estimated vision correction power is equal to the estimated value of the magnitude or to a modified value determined to be between said measured value of the magnitude and said estimated value of the magnitude.

When said magnitude is the refractive feature of the eye, the estimated vision correction power may be deduced from the estimated value of the magnitude by determining the vision correction power of the vision correction lens adapted to improve the visual performance of the eye having the estimated value of the magnitude. The distance between the vision correction lens and the eye is in particular taken into account. Alternatively, the estimated vision correction power may be determined to be between said measured vision correction power and said estimated vision correction power determined as mentioned above.

In an embodiment, said one or more memories also have in memory:
- a measured value Vmeas2 of a magnitude representative of another refractive feature of the eye of the individual, measured using said first optometry instrument,
- an estimated value Vest2 of said magnitude representative of another refraction feature of said eye of the individual, calculated using said model of the second optometry instrument.

This may mean a measured value of another vision correction power of said corrective lens to be placed in front of the eye of the individual or a measured value of another refractive feature of the eye of the individual, measured using said first optometry instrument and an estimated value of said other vision correction power of said eye of the individual or of said other refractive feature, calculated using said model of the second optometry instrument.

Said measured value Vmeas2 and said estimated value Vest2 of said magnitude representative of another refractive feature of the eye of the individual are recorded (block 50 of the single figure) in said one or more memories 100.

In step c), when the difference D2 between said measured value Vmeas1 and said estimated value Vest1 of said magnitude initially taken into account during steps a) and b) is less than the second threshold value T2 during step b), in other words when D2 = Vmeas1-Vest1 < T2 during step b), the one or more processors 200 are programmed to compare, in an additional step, (block 80 of the single figure) the difference D3 between said measured value Vmeas2 and said estimated value Vest2 of said magnitude representative of said other refraction feature to a third threshold value T3.

The one or more processors 200 determine said final value of the vision correction power taking into account this comparison.

When the difference D3 between said measured value Vmeas2 and said estimated value Vest2 of said magnitude representative of said other refraction feature is less than said third threshold value T3, in other words when D3 = Vmeas2-Vest2 < T3, the final value of the vision correction power is determined to be equal to a vision correction power corresponding to said estimated value of said magnitude representative of the other refractive feature or to a modified value determined to be between a vision correction power corresponding to said measured value of said magnitude representative of the other refractive feature and said estimated value of this magnitude (block 93).

In other words, the final value of the vision correction power may be determined equal to the estimated value of the other vision correction power or to the value of the vision correction power corresponding to the estimated value of the other refractive feature of the eye. Alternatively, it is determined to be equal to the modified value between said measured and estimated other vision correction power or between said values of the vision correction power corresponding to the measured and estimated values of the other refractive feature.

Preferably, the final value of the vision correction power may also comprise the estimated value of the vision correction power or the value of the vision correction power corresponding to the estimated value of the refractive feature of the eye initially taken into account during steps a) and b). It may also comprise the modified value between said measured and estimated vision correction power or between said values of the vision correction power corresponding to the measured and estimated values of the refractive feature.

Preferably, the one or more processors are programmed to determine the final values of all the vision correction powers of the corrective lens as having their estimated or modified values once one final value of vision correction power is determined to be equal to its estimated value.

When the difference D3 between said measured value Vmeas2 and said estimated value Vest2 of said magnitude representative of said other refractive feature is greater than or equal tosaid third threshold value T3, in other words when D3 = Vmeas2-Vest1 ≥ T3, the final value of the vision correction power is determined to be equal to a vision correction power corresponding to said measured value of this magnitude representative of said other refractive feature (block 94 of the single figure).

In other words, the final value of the vision correction power is determined to be equal to the measured value of the other vision correction power or to the value of the vision correction power corresponding to the measured value of the other refractive feature of the eye.

As mentioned above, preferably, the final value of the vision correction power may also comprise the measured value of the vision correction power or the value of the vision correction power corresponding to the measured value of the refractive feature of the eye initially taken into account during steps a) and b).

Preferably, the one or more processors are programmed to determine the final values of all the vision correction powers of the corrective lens as having their measured values once one final value of vision correction power is determined to be equal to its measured value.

A sensitivity of the individual to a variation of said vision correction power and/or other vision correction power is recorded in said one or more memories and said one or more processors are programmed to take into account said sensitivity when determining the final value. This may be achieved by modifying the threshold value according to the sensitivity, for example by using a threshold value proportional to the sensitivity.

Said one or more memories 100 may also have in memory measured values of a plurality of refraction features of an eye of the individual and/or corresponding vision correction powers, measured using a first optometry instrument ; estimated values of said plurality of refraction features of the eye of the individual and/or of the corresponding vision correction powers, calculated using a model of a second optometry instrument, and current values of said refractive features of the eye and/or of said vision correction powers of a current corrective lens previously worn by the individual, each refractive feature and/or corresponding vision correction power of said plurality of refractive features and vision correction powers being associated with specific first, second and third threshold values and said one or more processors being programmed to achieve steps a) to c) for each refractive feature of said plurality of refractive features, taking into account the associated threshold values.

Said one or more memories may also have in memory the subjective appreciation or visual acuity obtained with said vision correction powers.

Said refractive feature comprises at least one of the following: spherical refraction, cylindrical refraction and axis, spherical equivalent power, addition, J0, J45.

Said vision correction power of the vision correction lens comprise at least one of the following: spherical power, cylindrical power and axis of the vision correction lens. It may also comprise addition, spherical equivalent power, J0, J45 at one or several points of the lens.

Additionally, the one or more processors may be programmed to determine additional trials for the subjective test performed to determine the refractive features of the eye of the individual and/or the vision correction powers of the vision correction lens adapted to the eye of the individual based on the comparisons performed according to the device and method of the invention.

In the following, several examples of implementation of the device and method according to the invention will be described.

In an embodiment, the relevant personal parameters determined statistically are age and type of ametropia.

For young individuals, for example individuals whose age is below 40 years old, the relevant magnitudes determined statistically are the spherical power or the spherical equivalent power and the cylindrical power component J45 of the vision correction lens.

For presbyopic individuals, usually individuals with age above 40 or with a non-null addition, the relevant magnitudes determined statistically are the spherical or spherical equivalent power, the addition and the cylindrical power component J45 of the vision correction lens.

Corresponding values of first and second thresholds have been statistically determined in relation with these magnitudes and personal parameters.

The thresholds define the limits beyond which there is a high risk of rejection of the vision correction lens to be used in a new optical equipment by the individual.

Preferably, the relevant magnitudes, steps and thresholds are determined taking into account the sensitivity of the individual.

For young individuals, the first and second thresholds associated with the spherical power of the vision correction lens are equal to 0.265 diopter and 0.34 diopter (D) respectively. The third threshold associated with the cylindrical power component J45 is equal to -0.25 diopter. This third threshold associated with the cylindrical power component J45 may also be rounded to zero. These first, second and third thresholds are recorded in said one or more memories 100.

The one or more memories 100 of the device according to the invention have in memory:
- an initial measured value of the spherical equivalent power of the vision correction lens for correcting the eye of the individual measured using a first optometry instrument such as a very high precision subjective refraction device providing a spherical equivalent power with a precision comprised between 0.01 and 0.24 diopter,
- an estimated value of said spherical equivalent power calculated using a predetermined model of a second optometry instrument such as a classical subjective refraction device with a phoropter having a precision of 0.25 diopter,
- a current value of said spherical equivalent power corresponding to the spherical equivalent power of the current corrective lens worn by the individual before the implementation of the device of the invention,
- a measured value of the cylindrical power component J45 of the vision correction lens for correcting the eye of the individual measured using said first optometry instrument,
- an estimated value of said cylindrical power component J45 of the vision correction lens for correcting the eye of the individual, calculated using said model of said second optometry instrument.

The cylindrical power component J45 is here a magnitude representative of another refractive feature of the eye as defined above.

When the individual is young, the one or more processors of the device according to the invention are programmed to first compare a difference between the measured value and the current value of the spherical equivalent power to the first threshold value of 0.265 diopter (D).

When the difference between the measured spherical equivalent power and the current spherical equivalent power of the vision correction lens currently used by the individual is strictly below 0.265 D, the final spherical equivalent power for the new vision correction lens of the individual is determined to be equal to the measured spherical equivalent power.

This means that the measured and current value are sufficiently close for the individual to easily adapt to the new vision correction lens. The risk of the individual rejecting the new lens is small. The vision correction lens as a whole having all the measured values of the vision correction powers is chosen for the individual.

When the difference between the measured value and the current value of said spherical equivalent power is greater than or equal to said first threshold value, here greater than or equal to 0.265 D, the one or more processors are programmed to compare the difference between said measured value and said estimated value of the spherical equivalent power to the second threshold value, here equal to 0.34 D.

When the difference between the measured value and the estimated value of the spherical equivalent power is strictly below 0.34 D, the one or more processors 200 are programmed to compare the difference between the measured value and estimated value of the cylindrical power component J45 to the third threshold, which is equal to -0.025 D (and may be rounded to zero).

When the difference between the measured value and the estimated value of the cylindrical power component J45 is strictly below -0.025 D (or zero when rounded), the final vision correction powers, here the spherical equivalent power and the cylindrical power component J45 are determined to be equal to their estimated values.

Preferably, the vision correction lens as a whole having all the estimated values of the vision correction powers is determined for the individual by the device according to the invention.

When the difference between the measured value and the estimated value of the cylindrical power component J45 is greater than or equal to-0.025 D (or zero when rounded), the final vision correction powers, here the spherical equivalent power and the cylindrical power component J45 are determined to be equal to their measured values. The vision correction lens as a whole having all the measured values of the vision correction powers is determined by the device according to the invention for the individual. A correction of the measured sphere value may be considered, preferably the same correction for both eyes.

When the difference between the measured value and the estimated value of the spherical equivalent power is greater than or equal to0.34 D, the one or more processors 200 are programmed to determine the final spherical equivalent power of the vision correction lens as being equal to the estimated value of the spherical equivalent power.

Preferably, the vision correction lens as a whole having all the estimated values of the vision correction powers is determined for the individual by the device according to the invention. During a test of the above described device and method according to the invention on 30 young individuals, the estimated vision correction lens was determined 13 times among which the estimated vision correction lens was effectively preferred by the young individual 10 times (the measured vision correction lens being preferred 3 times) and the measured vision correction lens was determined 17 times, among which it was preferred 13 times by the young individual ( the estimated vision correction lens being preferred 4 times).

For presbyopic individuals, similar comparison steps a) and b) are performed for the spherical equivalent power. Different first and second thresholds may be determined.

A third threshold associated with the addition of the vision correction lens may be equal to 0, meaning that the measured and estimated values of addition may be directly compared.

The one or more memories 100 of the device according to the invention have in memory for presbyopic individuals:
- said initial measured value, estimated and current values of the spherical equivalent power of the vision correction lens, as defined above for young individuals,
- a measured value of the addition of the vision correction lens for correcting the eye of the individual measured using said first optometry instrument,
- an estimated value of said addition of the vision correction lens for correcting the eye of the individual, calculated using said model of said second optometry instrument,
- a current value of said addition corresponding to the addition of the current corrective lens worn by the individual before the implementation of the device of the invention, and optionally,
- said measured and estimated values of the cylindrical power component J45 of the vision correction lens for correcting the eye of the individual as defined for young individuals and a current value corresponding to the cylindrical power component J45 of the current corrective lens worn by the individual before the implementation of the device of the invention.

For presbyopic individuals, the one or more processors of the device according to the invention are programmed to first compare a difference between the measured value and the current value of the spherical equivalent power to the first threshold value determined for presbyopic individuals.

When the difference between the measured spherical equivalent power and the current spherical equivalent power of the vision correction lens currently used by the individual is strictly less than, in other words below, the first threshold, the final spherical equivalent power for the new vision correction lens of the individual is determined to be equal to the measured spherical equivalent power.

When the difference between the measured value and the current value of said spherical equivalent power is greater than or equal to, in other words, above or equal to, said first threshold value, the one or more processors are programmed to compare the difference between said measured value and said estimated value of the spherical equivalent power to the second threshold for presbyopic individuals.

Based on the result of this comparison between the difference between the measured value and the estimated value of the spherical equivalent power and the second threshold for presbyopic individuals, the one or more processors 200 are programmed to compare the difference between the measured value and estimated value of the addition to the third threshold for presbyopic individuals and/or to determine the final value of the spherical equivalent power to be the measured or estimated value.

Additionally, for presbyopic individuals, in the cases where the final spherical equivalent power determined is different from the measured spherical equivalent power, the addition should preferably be retested. The one or more processors may be programmed to issue an alert requiring to test the addition again.

Moreover, for presbyopic individuals, the one or more processors may be programmed to compare the difference between the measured value and the current value of the cylindrical power component J45 to a predetermined threshold value and when said difference is over said predetermined threshold value, compare the difference between the measured value and estimated value of the cylindrical power component J45 to another predetermined threshold value.

When the measured value of the cylindrical power component J45 is different from the current value of the cylindrical power component J45, or when the difference between the measured value and the estimated value of the cylindrical power component J45 is over said other predetermined threshold value, the one or more processors may be programmed to issue a recommendation to test J45 again. The subjective test of astigmatism from which the value of J45 was obtained is then rerun.

Said final value of the cylindrical power component J45 is determined based on said additional test.

## Claims

1. Device for determining a final value of a vision correction power of a final corrective lens to be placed in front of an eye of an individual, said device comprising a computer with one or more memories and one or more processors, wherein :
- said one or more memories have in memory:
- an initial measured value of a magnitude representative of a refractive feature of the eye of the individual, measured using a first optometry instrument,
- an estimated value of said magnitude, calculated using a predetermined model of a second optometry instrument,
- a current value of said magnitude determined based on a current corrective lens previously worn by the individual,
- said one or more processors are programmed to achieve the following steps:
a) comparing a difference between the measured value and the current value of said magnitude to a first threshold value, and
b) when the difference between the measured value and the current value of said magnitude is greater than or equal to said first threshold value, comparing the difference between said measured value and said estimated value of said magnitude to a second threshold value,
c) determining said final value of the vision correction power based on said comparisons.

2. Device according to claim 1, wherein said one or more memories have in memory data relative to at least one relevant personal parameter relative to said individual and said first and second threshold value are determined taking into account said data.

3. Device according to claim 2, wherein said magnitude representative of a refractive feature of an eye of the individual is determined taking into account said data relative to at least one relevant personal parameter relative to said individual.

4. Device according to any one of claims 2 or 3, wherein said relevant personal parameter comprises at least one of age, gender, sensitivity to a variation of said vision correction power, kind of ametropia, features of a previous vision correction equipment used, one or more values of a refractive feature of the eye determined through an objective method, type of complaints of said individual relative to his previous vision correction equipment, design of the current/final vision correction lens.

5. Device according to any one of claims 2 to 4, wherein said relevant personal parameters are determined statistically on the basis of a database comprising data relative to a set of personal parameters and corresponding one or more measured values of a vision correction power of a lens and/or one or more values of a refractive feature of an eye determined with first and second optometry instruments, a current value of vision correction power provided by a current corrective lens worn by the individual and/or a current value of said refractive feature, for a group of test individuals, and preferred value of the vision correction power chosen by each test individual, by determining correlations between personal parameters of said set and the preferred value of vision correction power.

6. Device according to claim 5, wherein said magnitude and said first and second thresholds are determined statistically based on said database, by determining correlations between the values of said relevant personal parameters and the preferred value of vision correction power.

7. Device according to any one of claims 1 to 6, wherein, in step c), when the difference between the measured value and the current value of said magnitude is less than said first threshold value during step a), the one or more processors are programmed to determine the final value of vision correction power as equal to a measured vision correction power corresponding to the measured value of said magnitude.

8. Device according to any one of claims 1 to 7, wherein, in step c), when the difference between said measured value and said estimated value of said magnitude is greater than or equal to the second threshold value during step b), the one or more processors are programmed to determine the final value of vision correction power as equal to an estimated vision correction power corresponding to the estimated value of said magnitude or a modified value determined to be between a measured vision correction power corresponding to the measured value of said magnitude and said estimated vision correction power.

9. Device according to any one of claims 1 to 8, wherein, said one or more memories having in memory:
- a measured value of a magnitude representative of another refractive feature of the eye of the individual, measured using said first optometry instrument,
- an estimated value of said magnitude representative of another refraction feature of said eye of the individual, calculated using said model of the second optometry instrument,
in step c), when the difference between said measured value and said estimated value of said magnitude representative of another refractive feature is less than the second threshold value during step b), the one or more processors are programmed to compare the difference between said measured value and said estimated value of said magnitude representative of said other refraction feature to a third threshold value and
determine said final value of the vision correction power taking into account this comparison.

10. Device according to claim 9, wherein, when the difference between said measured value and said estimated value of said magnitude representative of said other refraction feature is less than said third threshold value, the final value of the vision correction power is determined to be equal to a vision correction power corresponding to said estimated value of said magnitude representative of the other refractive feature or a modified value determined to be between a vision correction power corresponding to said measured value of said magnitude representative of the other refractive feature and said estimated value of this magnitude.

11. Device according to any one of claims 9 and 10, wherein, when the difference between said measured value and said estimated value of said magnitude representative of said other refractive feature is greater than or equal to said third threshold value, the final value of the vision correction power is determined to be equal to a vision correction power corresponding to said measured value of this magnitude.

12. Device according to any one of claims 1 to 11, wherein, a sensitivity of the individual to a variation of said vision correction power is recorded in said one or more memories and said one or more processors are programmed to take into account said sensitivity when determining the final value.

13. Device according to any one of claims 1 to 12, wherein said one or more memories have in memory measured values of a plurality of refraction features of an eye of the individual and/or corresponding vision correction powers, measured using a first optometry instrument ; estimated values of said plurality of refraction features of the eye of the individual and/or of the corresponding vision correction powers, calculated using a model of a second optometry instrument, and current values of said refractive features of the eye and/or of said vision correction powers of a current corrective lens previously worn by the individual,
- each refractive feature and/or corresponding vision correction power of said plurality of refractive features and vision correction powers being associated with specific first, second and third threshold values and said one or more processors being programmed to achieve steps a) to c) for each refractive feature of said plurality of refractive features, taking into account the associated threshold values.

14. Device according to any of claims 1 to 13, wherein said refractive feature comprises at least one of the following: sphere, cylinder, spherical equivalent power, addition, J0, J45.

15. Method for determining a final value of a vision correction power of a final corrective lens to be place in front of an eye of an individual, comprising the following steps:
- recording in one or more memories of a computer:
- an initial measured value of a magnitude representative of a refractive feature of the eye of the individual, measured using a first optometry instrument,
- an estimated value of said magnitude, calculated using a predetermined model of a second optometry instrument,
- a current value of said magnitude determined based on a current corrective lens previously worn by the individual,
- thanks to one or more processors of said computer :
a) comparing a difference between the measured value and the current value of said magnitude to a first threshold value, and
b) when the difference between the measured value and the current value of said magnitude is greater than or equal to said first threshold value, comparing the difference between said measured value and said estimated value of said magnitude to a second threshold value,
c) determining said final value of the vision correction power based on said comparisons.

## Patentansprüche

1. Vorrichtung zum Bestimmen eines endgültigen Wertes einer Sehkorrekturstärke einer endgültigen Korrekturlinse, die vor einem Auge einer Person angeordnet sein soll, wobei die Vorrichtung einen Computer mit einem oder mehreren Speichern und einem oder mehreren Prozessoren umfasst, wobei:
- der eine oder die mehreren Speicher Folgendes im Speicher aufweisen:
- einen Ausgangsmesswert einer Größe, die eine Brechungseigenschaft des Auges der Person darstellt, der unter Verwendung eines ersten Optometrieinstruments gemessen wurde,
- einen Schätzwert der Größe, der unter Verwendung eines vorgegebenen Modells eines zweiten Optometrieinstruments berechnet wurde,
- einen aktuellen Wert der Größe, der auf der Grundlage einer aktuellen Korrekturlinse, die vorher von der Person getragen wurde, bestimmt wurde,
- der eine oder die mehreren Prozessoren programmiert sind, die folgenden Schritte auszuführen:
a) Vergleichen einer Differenz zwischen dem Messwert und dem aktuellen Wert der Größe mit einem ersten Schwellenwert, und
b) dann, wenn die Differenz zwischen dem Messwert und dem aktuellen Wert der Größe größer oder gleich dem ersten Schwellenwert ist, Vergleichen der Differenz zwischen dem Messwert und dem Schätzwert der Größe mit einem zweiten Schwellenwert,
c) Bestimmen des endgültigen Wertes der Sehkorrekturstärke auf der Grundlage der Vergleiche.

2. Vorrichtung nach Anspruch 1, wobei der eine oder die mehreren Speicher im Speicher Daten bezüglich mindestens eines relevanten persönlichen Parameters bezüglich der Person aufweisen und der erste und der zweite Schwellenwert unter Berücksichtigung der Daten bestimmt werden.

3. Vorrichtung nach Anspruch 2, wobei die Größe, die eine Brechungseigenschaft eines Auges der Person darstellt, unter Berücksichtigung der Daten bezüglich des mindestens einen relevanten persönlichen Parameters bezüglich der Person bestimmt wird.

4. Vorrichtung nach einem der Ansprüche 2 oder 3, wobei der relevante persönliche Parameter das Alter und/oder das Geschlecht und/oder eine Empfindlichkeit für eine Veränderung der Sehkorrekturstärke und/oder eine Art der Fehlsichtigkeit und/oder Eigenschaften einer vorher verwendeten Sehkorrekturausstattung und/oder einen oder mehrere Werte einer Brechungseigenschaft des Auges, die durch ein Objektivverfahren bestimmt wurden, und/oder Arten von Beschwerden der Person bezüglich ihrer vorherigen Sehkorrekturausstattung und/oder einen Entwurf der aktuellen/endgültigen Sehkorrekturlinse umfasst.

5. Vorrichtung nach einem der Ansprüche 2 bis 4, wobei die relevanten persönlichen Parameter auf der Grundlage einer Datenbank, die Daten umfasst, die sich auf einen Satz persönlicher Parameter beziehen und einem oder mehreren Messwerten einer Sehkorrekturstärke einer Linse und/oder einem oder mehreren Werten einer Brechungseigenschaft eines Auges, die mit dem ersten und dem zweiten Optometrieinstrument bestimmt wurden, einem aktuellen Wert einer Sehkorrekturstärke, der durch eine aktuelle Korrekturlinse bereitgestellt wird, die von der Person getragen wird, und/oder einem aktuellen Wert der Brechungseigenschaft für eine Gruppe von Testpersonen und einem bevorzugten Wert der Sehkorrekturstärke, der von jeder Testperson gewählt wurde, entsprechen, durch Bestimmen von Korrelationen zwischen persönlichen Parametern des Satzes und dem bevorzugten Wert der Sehkorrekturstärke statistisch bestimmt werden.

6. Vorrichtung nach Anspruch 5, wobei die Größe und der erste und der zweite Schwellenwert auf der Grundlage der Datenbank durch Bestimmen von Korrelationen zwischen den Werten der relevanten persönlichen Parameter und dem bevorzugten Wert der Sehkorrekturstärke statistisch bestimmt werden.

7. Vorrichtung nach einem der Ansprüche 1 bis 6, wobei in Schritt c) dann, wenn während Schritt a) die Differenz zwischen dem Messwert und dem aktuellen Wert der Größe kleiner als der erste Schwellenwert ist, der eine oder die mehreren Prozessoren programmiert sind, den endgültigen Wert der Sehkorrekturstärke als gleich einer gemessenen Sehkorrekturstärke zu bestimmen, die dem Messwert der Größe entspricht.

8. Vorrichtung nach einem der Ansprüche 1 bis 7, wobei in Schritt c) dann, wenn während Schritt b) die Differenz zwischen dem Messwert und dem Schätzwert der Größe größer oder gleich dem zweiten Schwellenwert ist, der eine oder die mehreren Prozessoren programmiert sind, den endgültigen Wert der Sehkorrekturstärke als gleich einer geschätzten Sehkorrekturstärke, die dem Schätzwert der Größe entspricht, oder einem abgewandelten Wert, der derart bestimmt wird, dass er zwischen einer gemessenen Sehkorrekturstärke, die dem Messwert der Größe entspricht, und der geschätzten Sehkorrekturstärke liegt, zu bestimmen.

9. Vorrichtung nach einem der Ansprüche 1 bis 8, wobei der eine oder die mehreren Speicher Folgendes im Speicher aufweisen:
- einen Messwert einer Größe, die eine weitere Brechungseigenschaft des Auges der Person darstellt, der unter Verwendung des ersten Optometrieinstruments gemessen wurde,
- einen Schätzwert der Größe, die eine weitere Brechungseigenschaft des Auges der Person darstellt, der unter Verwendung des Modells des zweiten Optometrieinstruments berechnet wurde,
wobei in Schritt c) dann, wenn während Schritt b) die Differenz zwischen dem Messwert und dem Schätzwert der Größe, die eine weitere Brechungseigenschaft darstellt, kleiner als der zweite Schwellenwert ist, der eine oder die mehreren Prozessoren programmiert sind, die Differenz zwischen dem Messwert und dem Schätzwert der Größe, die die andere Brechungseigenschaft darstellt, mit einem dritten Schwellenwert zu vergleichen, und
den endgültigen Wert der Sehkorrekturstärke unter Berücksichtigung dieses Vergleichs zu bestimmen.

10. Vorrichtung nach Anspruch 9, wobei dann, wenn die Differenz zwischen dem Messwert und dem Schätzwert der Größe, die die andere Brechungseigenschaft darstellt, kleiner als der dritte Schwellenwert ist, der endgültige Wert der Sehkorrekturstärke derart bestimmt wird, dass er gleich einer Sehkorrekturstärke, die dem Schätzwert der Größe, die die andere Brechungseigenschaft darstellt, entspricht, oder einem abgewandelten Wert, der derart bestimmt wird, dass er zwischen einer Sehkorrekturstärke, die dem Messwert der Größe, die der anderen Brechungseigenschaft entspricht, und dem Schätzwert dieser Größe liegt, ist.

11. Vorrichtung nach einem der Ansprüche 9 und 10, wobei dann, wenn die Differenz zwischen dem Messwert und dem Schätzwert der Größe, die die andere Brechungseigenschaft darstellt, größer oder gleich dem dritten Schwellenwert ist, der endgültige Wert der Sehkorrekturstärke derart bestimmt wird, dass er gleich einer Sehkorrekturstärke ist, die dem Messwert dieser Größe entspricht.

12. Vorrichtung nach einem der Ansprüche 1 bis 11, wobei eine Empfindlichkeit der Person für eine Veränderung der Sehkorrekturstärke in dem einen oder den mehreren Speichern aufgezeichnet wird und der eine oder die mehreren Prozessoren programmiert sind, die Empfindlichkeit zu berücksichtigen, wenn der endgültige Wert bestimmt wird.

13. Vorrichtung nach einem der Ansprüche 1 bis 12, wobei der eine oder die mehreren Speicher im Speicher Messwerte mehrerer Brechungseigenschaften eines Auges der Person und/oder entsprechende Sehkorrekturstärken, die unter Verwendung eines ersten Optometrieinstruments gemessen wurden; Schätzwerte der mehreren Brechungseigenschaften des Auges der Person und/oder der entsprechenden Sehkorrekturwerte, die unter Verwendung eines Modells eines zweiten Optometrieinstruments berechnet wurden; und aktuelle Werte der Brechungseigenschaften des Auges und/oder der Sehkorrekturstärken einer aktuellen Korrekturlinse, die vorher von der Person getragen wurde, aufweisen,
- wobei jede Brechungseigenschaft und/oder entsprechende Sehkorrekturstärke der mehreren Brechungseigenschaften und Sehkorrekturstärken einem spezifischen ersten, zweiten und dritten Schwellenwert zugeordnet werden und der eine oder die mehreren Prozessoren programmiert sind, die Schritte a) bis c) für jede Brechungseigenschaft der mehreren Brechungseigenschaften unter Berücksichtigung der zugeordneten Schwellenwerte auszuführen.

14. Vorrichtung nach einem der Ansprüche 1 bis 13, wobei die Brechungseigenschaft mindestens ein Element der Folgenden umfasst: Sphäre, Zylinder, sphärisches Äquivalent, Zusatz, J0, J45.

15. Verfahren zum Bestimmen eines endgültigen Wertes einer Sehkorrekturstärke einer endgültigen Korrekturlinse, die vor einem Auge einer Person angeordnet sein soll, das die folgenden Schritte umfasst:
- Aufzeichnen in einem oder mehreren Speichern eines Computers:
- einen Ausgangsmesswert einer Größe, die eine Brechungseigenschaft des Auges der Person darstellt, der unter Verwendung eines ersten Optometrieinstruments gemessen wurde,
- einen Schätzwert der Größe, der unter Verwendung eines vorgegebenen Modells eines zweiten Optometrieinstruments berechnet wurde,
- einen aktuellen Wert der Größe, der auf der Grundlage einer aktuellen Korrekturlinse, die vorher von der Person getragen wurde, bestimmt wurde,
- durch den einen oder die mehreren Prozessoren des Computers:
a) Vergleichen einer Differenz zwischen dem Messwert und dem aktuellen Wert der Größe mit einem ersten Schwellenwert, und
b) dann, wenn die Differenz zwischen dem Messwert und dem aktuellen Wert der Größe größer oder gleich dem ersten Schwellenwert ist, Vergleichen der Differenz zwischen dem Messwert und dem Schätzwert der Größe mit einem zweiten Schwellenwert,
c) Bestimmen des endgültigen Wertes der Sehkorrekturstärke auf der Grundlage der Vergleiche.

## Revendications

1. Dispositif destiné à déterminer une valeur finale d'une puissance de correction de la vision d'une lentille correctrice finale devant être placée devant un œil d'un individu, ledit dispositif comprenant un ordinateur avec une ou plusieurs mémoires et un ou plusieurs processeurs, dans lequel :
- ladite ou lesdites mémoires ont en mémoire :
- une valeur mesurée initiale d'une grandeur représentative d'une caractéristique de réfraction de l'œil de l'individu, mesurée au moyen d'un premier instrument d'optométrie,
- une valeur estimée de ladite grandeur, calculée au moyen d'un modèle prédéterminé d'un deuxième instrument d'optométrie,
- une valeur actuelle de ladite grandeur déterminée sur la base d'une lentille correctrice actuelle précédemment portée par l'individu,
- ledit ou lesdits processeurs sont programmés pour réaliser les étapes suivantes :
a) comparaison d'une différence entre la valeur mesurée et la valeur actuelle de ladite grandeur à une première valeur seuil, et
b) lorsque la différence entre la valeur mesurée et la valeur actuelle de ladite grandeur est supérieure ou égale à ladite première valeur seuil, comparaison de la différence entre ladite valeur mesurée et ladite valeur estimée de ladite grandeur à une deuxième valeur seuil,
c) détermination de ladite valeur finale de la puissance de correction de la vision sur la base desdites comparaisons.

2. Dispositif selon la revendication 1, dans lequel ladite ou lesdites mémoires ont en mémoire des données relatives à au moins un paramètre personnel pertinent relatif audit individu et lesdites première et deuxième valeurs seuils sont déterminées en tenant compte desdites données.

3. Dispositif selon la revendication 2, dans lequel ladite grandeur représentative d'une caractéristique de réfraction d'un œil de l'individu est déterminée en tenant compte desdites données relatives à au moins un paramètre personnel pertinent relatif audit individu.

4. Dispositif selon l'une quelconque des revendications 2 et 3, dans lequel ledit paramètre personnel pertinent en comprend au moins un parmi l'âge, le sexe, la sensibilité à une variation de ladite puissance de correction de la vision, le type d'amétropie, des caractéristiques d'un équipement de correction de la vision précédent utilisé, une ou plusieurs valeurs d'une caractéristique de réfraction de l'œil déterminées par un procédé objectif, le type de reproches dudit individu concernant son équipement de correction de la vision précédent, la conception de la lentille de correction de la vision actuelle/finale.

5. Dispositif selon l'une quelconque des revendications 2 à 4, dans lequel lesdits paramètres personnels pertinents sont déterminés statistiquement à partir d'une base de données comprenant des données relatives à un ensemble de paramètres personnels et une ou plusieurs valeurs mesurées correspondantes d'une puissance de correction de la vision d'une lentille et/ou une ou plusieurs valeurs d'une caractéristique de réfraction d'un œil déterminées avec de premier et deuxième instruments d'optométrie, une valeur actuelle de puissance de correction de la vision fournie par une lentille correctrice actuelle portée par l'individu et/ou une valeur actuelle de ladite caractéristique de réfraction, pour un groupe d'individus témoins, et une valeur préférée de la puissance de correction de la vision choisie par chaque individu témoin, par détermination de corrélations entre des paramètres personnels dudit ensemble et la valeur préférée de puissance de correction de la vision.

6. Dispositif selon la revendication 5, dans lequel ladite grandeur et lesdits premier et deuxième seuils sont déterminés statistiquement à partir de ladite base de données, par détermination de corrélations entre les valeurs desdits paramètres personnels pertinents et la valeur préférée de puissance de correction de la vision.

7. Dispositif selon l'une quelconque des revendications 1 à 6 dans lequel, à l'étape c), lorsque la différence entre la valeur mesurée et la valeur actuelle de ladite grandeur est inférieure à ladite première valeur seuil lors de l'étape a), le ou les processeurs sont programmés pour déterminer la valeur finale de puissance de correction de la vision comme étant égale à une puissance de correction de la vision mesurée correspondant à la valeur mesurée de ladite grandeur.

8. Dispositif selon l'une quelconque des revendications 1 à 7 dans lequel, à l'étape c), lorsque la différence entre ladite valeur mesurée et ladite valeur estimée de ladite grandeur est supérieure ou égale à la deuxième valeur seuil lors de l'étape b), le ou les processeurs sont programmés pour déterminer la valeur finale de puissance de correction de la vision comme étant égale à une puissance de correction de la vision estimée correspondant à la valeur estimée de ladite grandeur ou une valeur modifiée déterminée comme se situant entre une puissance de correction de la vision mesurée correspondant à la valeur mesurée de ladite grandeur et ladite puissance de correction de la vision estimée.

9. Dispositif selon l'une quelconque des revendications 1 à 8, dans lequel ladite ou lesdites mémoires ont en mémoire :
- une valeur mesurée d'une grandeur représentative d'une autre caractéristique de réfraction de l'œil de l'individu, mesurée au moyen dudit premier instrument d'optométrie,
- une valeur estimée de ladite grandeur représentative d'une autre caractéristique de réfraction dudit œil de l'individu, calculée au moyen dudit modèle du deuxième instrument d'optométrie,
à l'étape c), lorsque la différence entre ladite valeur mesurée et ladite valeur estimée de ladite grandeur représentative d'une autre caractéristique de réfraction est inférieure à la deuxième valeur seuil lors de l'étape b), le ou les processeurs sont programmés pour comparer la différence entre ladite valeur mesurée et ladite valeur estimée de ladite grandeur représentative de ladite autre caractéristique de réfraction à une troisième valeur seuil et
déterminer ladite valeur finale de la puissance de correction de la vision en tenant compte de cette comparaison.

10. Dispositif selon la revendication 9 dans lequel, lorsque la différence entre ladite valeur mesurée et ladite valeur estimée de ladite grandeur représentative de ladite autre caractéristique de réfraction est inférieure à ladite troisième valeur seuil, la valeur finale de la puissance de correction de la vision est déterminée comme étant égale à une puissance de correction de la vision correspondant à ladite valeur estimée de ladite grandeur représentative de l'autre caractéristique de réfraction ou une valeur modifiée déterminée comme se situant entre une puissance de correction de la vision correspondant à ladite valeur mesurée de ladite grandeur représentative de l'autre caractéristique de réfraction et ladite valeur estimée de cette grandeur.

11. Dispositif selon l'une quelconque des revendications 9 et 10 dans lequel, lorsque la différence entre ladite valeur mesurée et ladite valeur estimée de ladite grandeur représentative de ladite autre caractéristique de réfraction est supérieure ou égale à ladite troisième valeur seuil, la valeur finale de la puissance de correction de la vision est déterminée comme étant égale à une puissance de correction de la vision correspondant à ladite valeur mesurée de cette grandeur.

12. Dispositif selon l'une quelconque des revendications 1 à 11, dans lequel une sensibilité de l'individu à une variation de ladite puissance de correction de la vision est enregistrée dans ladite ou lesdites mémoires et ledit ou lesdits processeurs sont programmés pour tenir compte de ladite sensibilité lors de la détermination de la valeur finale.

13. Dispositif selon l'une quelconque des revendications 1 à 12, dans lequel ladite ou lesdites mémoires ont en mémoire des valeurs mesurées d'une pluralité de caractéristiques de réfraction d'un œil de l'individu et/ou des puissances de correction de la vision correspondantes, mesurées au moyen d'un premier instrument d'optométrie, des valeurs estimées de ladite pluralité de caractéristiques de réfraction de l'œil de l'individu et/ou les puissances de correction de la vision correspondantes, calculées au moyen d'un modèle d'un deuxième instrument d'optométrie, et des valeurs actuelles desdites caractéristiques de réfraction de l'œil et/ou desdites puissances de correction de la vision d'une lentille correctrice actuelle précédemment portée par l'individu,
- chaque caractéristique de réfraction et/ou chaque puissance de correction de la vision correspondante de ladite pluralité de caractéristiques de réfraction et de puissances de correction de la vision étant associées à des première, deuxième et troisième valeurs seuils spécifiques et ledit ou lesdits processeurs étant programmés pour réaliser les étapes a) à c) pour chaque caractéristique de réfraction de ladite pluralité de caractéristiques de réfraction en tenant compte des valeurs seuils associées.

14. Dispositif selon l'une quelconque des revendications 1 à 13, dans lequel ladite caractéristique de réfraction en comprend au moins une des suivantes : sphère, cylindre, puissance sphérique équivalente, addition, J0, J45.

15. Procédé destiné à déterminer une valeur finale d'une puissance de correction de la vision d'une lentille correctrice finale devant être placée devant un œil d'un individu, comprenant les étapes suivantes :
- enregistrement dans une ou plusieurs mémoires d'un ordinateur :
- d'une valeur mesurée initiale d'une grandeur représentative d'une caractéristique de réfraction de l'œil de l'individu, mesurée au moyen d'un premier instrument d'optométrie,
- d'une valeur estimée de ladite grandeur, calculée au moyen d'un modèle prédéterminé d'un deuxième instrument d'optométrie,
- d'une valeur actuelle de ladite grandeur déterminée sur la base d'une lentille correctrice actuelle précédemment portée par l'individu,
- grâce à un ou plusieurs processeurs dudit ordinateur :
a) comparaison d'une différence entre la valeur mesurée et la valeur actuelle de ladite grandeur à une première valeur seuil, et
b) lorsque la différence entre la valeur mesurée et la valeur actuelle de ladite grandeur est supérieure ou égale à ladite première valeur seuil, comparaison de la différence entre ladite valeur mesurée et ladite valeur estimée de ladite grandeur à une deuxième valeur seuil,
c) détermination de ladite valeur finale de la puissance de correction visuelle sur la base desdites comparaisons.
